# EUROPÄISCHE PATENTSCHRIFT

(11) **EP 0 821 342 B1**
(45) Veröffentlichungstag und Bekanntmachung des Hinweises auf die Patenterteilung: **25.06.2003**
(21) Anmeldenummer: 97111385.7
(22) Anmeldetag: 05.07.1997
(51) Int. Cl.: G10K 9/12

(54) **Stosswellenquelle nach dem elektromagnetischen Prinzip**
Electromagnetic shockwave generator
Générateur d'onde de choc électromagnétique

(30) Priorität: 26.07.1996 DE 19630180
(43) Veröffentlichungstag der Anmeldung: 28.01.1998
(73) Patentinhaber: Dornier MedTech Systems GmbH, 82234 Wessling (DE)
(72) Erfinder: Eizenhöfer, Harald, Dipl.-Phys., 82229 Seefeld (DE)
(74) Vertreter: Grünecker, Kinkeldey, Stockmair & Schwanhäusser Anwaltssozietät

(56) Entgegenhaltungen:
- EP-A- 0 298 334
- DE-A- 3 443 295
- DE-A- 3 634 378

## Beschreibung

Die Erfindung betrifft eine Stoßwellenquelle nach dem elektromagnetischen Prinzip mit definierter Fokussierung, gemäß dem Oberbegriff des Patentanspruches 1.

Bei elektromagnetischen Stoßwellensystemen in sphärischer oder sonstiger Geometrie hängt der Wirkungsgrad entscheidend von der elektromagnetischen Kopplung von der Erregerspule zur leitfähigen Membran ab. Die gute Kopplung, d.h. das Anschmiegen der Membran an die Spulenwindungen, wird bei bekannten Systemen durch Überdruck vor der Membran bzw. durch Unterdruck im Zwischenraum zwischen Spule und Membran erreicht. Der erforderliche technische Aufwand, um diesen Unter- bzw. Überdruck aufzubauen, aufrechtzuerhalten und zu überwachen, ist entsprechend und erhöht die Herstellkosten sowie das Gewicht und das Volumen der Stoßwellenquelle.

Eine gattungsbildende Stoßwellenquelle ist bei einem ersten Ausführungsbeispiel gemäß der DE 33 12 014 A1 bekannt. Ein zweites Ausführungsbeispiel weist ein gummi- oder gelartiges Übertragungsmedium auf.

Aus der japanischen Veröffentlichung "Patent Abstracts of Japan, JP 1-317 431 (A), Sect. C-697, March 6, 1990 Vol. 14/No. 118" ist eine Stoßwellenquelle mit definierter Fokussierung bekannt, bei welcher die Spule aus einem elektrisch leitfähigen Draht mit rechteckigem Querschnitt gewickelt ist, um eine glatte Spulenoberfläche mit konstantem, kleinem Abstand zur Membran zu erzielen und dadurch den Wirkungsgrad der Anordnung zu erhöhen.

Die DE 36 34 378 A1 beschreibt eine elektromagnetische Stoßwellenquelle mit zwei parallel und deckungsgleich angeordneten Spulen, welche durch gleich- oder gegensinnigen Stromfluß sowohl anziehend als auch abstoßend aufeinander wirken können. Eine Spule ist relativ unbeweglich (gehäusefest), die andere relativ leicht beweglich (mit einer Membran verbunden). Die Anordnung soll die Erzeugung von Stoß-(Druck-) und Unterdruckwellen ermöglichen.

Aus der EP 0 298 334 A1 ist eine elektromagnetische Stoßwellenquelle bekannt, welche eine flexible, mit einer Vielzahl von elektrisch leitenden Plättchen gezielt belegte Membran aufweist. Über die Massenträgheit und Leitfähigkeit der Plättchen läßt sich der Stoßwellenverlauf örtlich gezielt beeinflussen, im Gegensatz zu einer üblichen, homogenen Metallmembran.

Eine Stoßwellenquelle bekannter Art ist beispielsweise auch in der DE-PS 34 43 295 beschrieben. Die eine Ausführung gemäß Fig. 1 benutzt als Übertragungsmedium eine Flüssigkeit, z.B. Wasser oder einen flüssigen Halogenkohlenwasserstoff, welche unter statischem Überdruck steht und dadurch die Metallmembran über eine dünne Isolierschicht gegen die im Gehäuse abgestützte, kugelkalottenförmige Spule drückt.

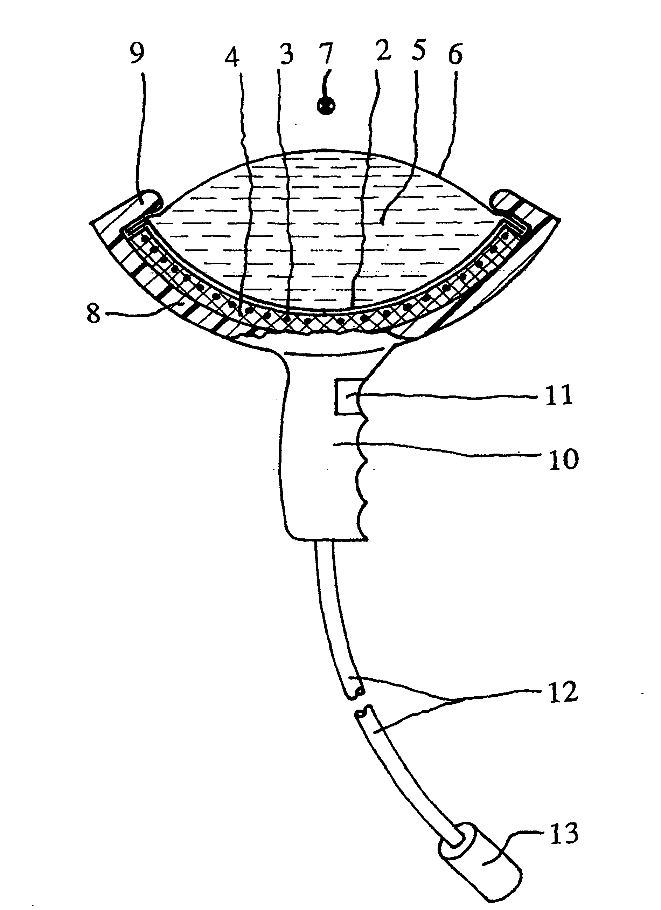

Die andere Ausführung nach Fig. 2 benutzt als Übertragungsmedium vor der Membran einen gummielastischen Festkörper, welcher ebenfalls unter statischem Überdruck steht und somit gegen die Membran drückt. Dies wird hier nicht durch eine äußere Druckquelle sondern durch die Installation des gummielastischen Körpers im komprimierten Zustand erreicht. Dafür ist eine Konstruktion erforderlich, welche den elastischen Festkörper auf einem Großteil seiner Oberfläche mit stabilen Wänden unter Druck hält. Hierbei sind teilweise Nachteile hinsichtlich Gewicht und Bauvolumen zu erwarten. Außerdem ist i.a. die Stoßwellenübertragung durch einen elastischen Körper nicht so effizient wie z.B. durch eine Flüssigkeit.

Der Erfindung liegt demgegenüber die Aufgabe zugrunde, eine Stoßwellenquelle nach dem elektromagnetischen Prinzip mit definierter Fokussierung, insbesondere einen handgeführten Therapiekopf, mit einer in einem Spulenträger angeordneten, zwischen einem Gehäuse und einer Membran befindlichen Spule und mit einem Übertragungsmedium zu schaffen, welche sich durch einen hohen Wirkungsgrad und gute Handlichkeit trotz geringer Herstellkosten auszeichnet.

Diese Aufgabe wird durch die im Anspruch 1 gekennzeichneten Merkmale gelöst, in Verbindung mit den gattungsbildenden Merkmalen in dessen Oberbegriff.

Die Spule ist in einen kompressiblen und auch anderweitig elastisch verformbaren Spulenträger eingebettet, welcher in komprimiertem Zustand unter Ausübung definierter Druckspannungen auf die Membran zwischen dieser und dem Gehäuse installiert ist. Die Membran und das Gehäuse sind in aller Regel ausreichend steif, um diesem Druck ohne relevante Verformungen standzuhalten. Dabei ist dem Fachmann klar, daß die Spule möglichst nahe an der der Membran zugewandten Oberfläche des Spulenträgers anzuordnen ist, unter Beachtung einer noch ausreichenden Verformungskapazität und Isolationswirkung dieses membrankontaktierenden Trägerbereiches. Auf diese Weise wird eine sehr gute elektromagnetische Kopplung Spule/Membran infolge der engen, gleichmäßigen mechanischen Anschmiegung erreicht, ohne nennenswerten Mehraufwand bezüglich Gewicht und Bauvolumen. Pneumatische bzw. hydraulische Subsysteme für die Über- bzw. Unterdruckerzeugung und die Drucküberwachung sowie -regelung sind nicht erforderlich. Der elastische Spulenträger paßt sich in relativ weiten Grenzen an variierende Krümmungsradien an und ist daher für therapeutisch unterschiedliche Stoßwellen-Therapieköpfe verwendbar.

Die Unteransprüche 2 bis 6 kennzeichnen bevorzugte Ausgestaltungen der Stoßwellenquelle nach dem Hauptanspruch.

Die Erfindung wird anschließend anhand der Zeichnung noch näher erläutert. Die Figur zeigt in vereinfachter, nicht maßstäblicher Wiedergabe eine kombinierte Schnitt-/Ansichtsdarstellung einer Stoßwellenquelle.

Die dargestellte Stoßwellenquelle 1 ist ein handgeführter Therapiekopf mit kurzem Fokusabstand (Fokus 7), welcher mehr für oberflächennahen Gebrauch (Orthopädie) als für tiefliegende Steinzertrümmerung geeignet ist. Das Gehäuse 8 der Stoßwellenquelle 1 ist mit einem Handgriff 10 versehen, in den ein Auslöseknopf 11 zur Aktivierung integriert ist. Das angedeutete Kabel 12 mit Stecker 13 wird an eine - nicht dargestellte - elektrische Versorgungseinheit angeschlossen.

Die Hauptfunktionselemente der Stoßwellenquelle 1 sind die kugelkalottenförmige, elektrisch leitende Membran 2 und die Spule 3, welche in einen elastischen Spulenträger 4 eingebettet ist. Mit der Membran 2 steht ein Übertragungsmedium 5, vorzugsweise Wasser oder Gel, in mechanischem Kontakt und wird nach außen, also zum Patienten hin, von einer stoßwellenleitenden Wand 6 begrenzt. Letztere kann als elastische Schutzfolie oder als elastischer Balg mit definierten, nachgiebigen Bereichen ausgeführt sein. Die Elemente 2, 4 und 6 sind im Gehäuse 8 mittels seines umlaufenden Randes 9 formschlüssig fixiert.

Es wird darauf hingewiesen, daß die Elemente 2, 4, 6 und 9 in der Figur aus Gründen der Erkennbarkeit bzw. Übersichtlichkeit mit geringem gegenseitigem Abstand dargestellt sind. In Wirklichkeit ist es jedoch so, daß die Membran 2 vollflächig am Spulenträger 4 anliegt, und daß die der Befestigung dienenden Randbereiche der Membran 2 und der Wand 6 sowohl aneinander als auch am Gehäuserand 9 bzw. am Spulenträger 4 anliegen. Wie bereits mehrfach erwähnt, ist der Spulenträger 4 in elastisch komprimiertem Zustand zwischen Membran 2 und Gehäuse 8 eingeschlossen, wodurch eine besonders gute mechanische und elektromagnetische Kopplung zur Membran 2 erzielt wird.

Es ist günstig, den Spulenträger 4 als ebenes, scheibenförmiges Bauteil konstanter Dicke mit ebener Spule herzustellen. Durch elastische Verwölbung und Kompression läßt sich ein derartiger Spulenträger 4 in unterschiedlichen Therapieköpfen mit verschiedenem Fokusabstand verwenden.

Das Gehäuse 8 der Stoßwellenquelle 1 ist bevorzugt als Spritzgußteil aus Hartkunststoff, ggf. mit Faserverstärkung, ausgeführt, was eine leichte und formstabile Bauweise ermöglicht. Die Elemente 2, 4 und 6 können nachträglich in das fertige Gehäuse 8 eingeclipst werden. Sie können aber auch bereits beim Spritzgießen in das Gießwerkzeug eingelegt werden, wobei die Wand 6 wiederum auch nachträglich - nach Befüllung mit Wasser oder Gel - installiert werden kann. Es bleibt noch anzumerken, daß Details wie der Gehäuserand 9, die Ränder der Elemente 2, 4 und 6 etc. in der Figur aus optischen Gründen übertrieben groß dargestellt sind. Dem Fachmann sind die tatsächlichen Dimensionen bzw. Relationen bekannt.

## Patentansprüche

1. Stoßwellenquelle (1) nach dem elektromagnetischen Prinzip mit definierter Fokussierung, insbesondere handgeführter Therapiekopf, mit einer elektrisch leitfähigen, räumlich gekrümmten Membran (2), mit einer zu dieser benachbarten, mit Stromimpulsen beaufschlagbaren Spule (3), mit einer schaltbaren elektrischen Verbindung (11, 12, 13) zwischen der Spule (3) und einer elektrischen Versorgungseinheit, mit einem die Membran (2) und die in einem eine definierte Druckspannung aufnehmenden Spulenträger (4) eingebettete Spule (3) aufnehmenden Gehäuse (8), zwischen dem und der Membran (2) sich örtlich der Spulenträger (4) befindet, sowie mit einem an die Membran (2) angrenzenden und auf diese Druck ausübenden Übertragungsmedium (5), **dadurch gekennzeichnet, daß** der Spulenträger (4) verformbar und elastisch kompressibel ausgeführt ist, daß der Spulenträger (4) in komprimiertem Zustand installiert ist, und daß nach der Montage die eingebaute Menge des Übertragungsmediums (5) während des Betriebszustandes unbeeinflußt bleibt.

2. Stoßwellenquelle nach Anspruch 1, **dadurch gekennzeichnet, daß** der Spulenträger (4) im unverformten, herstellungsbedingten Zustand ein ebenes, scheibenförmiges Bauteil konstanter Dicke mit darin eben angeordneter Spule (3) bildet.

3. Stoßwellenquelle nach Anspruch 1 oder 2, **dadurch gekennzeichnet, daß** die Membran (2) als tiefgezogene Metallkalotte ausgeführt ist.

4. Stoßwellenquelle nach einem oder mehreren der Ansprüche 1 bis 3, **dadurch gekennzeichnet, daß** ein als flüssiges oder gelförmiges Medium ausgebildetes Übertragungsmedium (5) nach außen von einer Wand (6) begrenzt wird, welche als Schutzfolie oder Balg ausgeführt ist.

5. Stoßwellenquelle nach einem oder mehreren der Ansprüche 1 bis 4, **dadurch gekennzeichnet, daß** das Gehäuse (8) als Hartkunststoffteil mit angeformtem Handgriff (10) und integriertem Auslöseknopf (11), vorzugsweise als Spritzgußteil, ausgeführt ist.

6. Stoßwellenquelle nach einem oder mehreren der Ansprüche 4 oder 5 **dadurch gekennzeichnet, daß** der Spulenträger (4), die Membran (2) und die das Übertragungsmedium (5) nach außen begrenzende Wand (6) formschlüssig und demontierbar in dem Gehäuse (8) gehalten sind.

## Claims

1. Shock wave source (1) on the electromagnetic principle with defined focusing, particularly hand-held therapy head, with an electrically conducting, spatially crooked membrane (2), with adjacent to it a coil (3) to which current pulses can be applied, with a switchable electrical connection (11, 12, 13) between the coil (3) and an electrical supply unit, with a housing (8) which receives the membrane (2) and the coil (3) which is embedded in a coil support (4) which receives a defined compressive strain, between which housing (8) and the membrane (2) the coil support (4) is located, and a propagation medium (5) which is adjacent to the membrane (2) and exerts pressure on it, **characterized in that** the coil support (4) is in deformable and elastically compressible form, that the coil support (4) is installed in the compressed state, and that after assembly the built-in quantity of the propagation medium (5) is not affected during the operating state.

2. Shock wave source according to Claim 1, **characterized in that** the coil support (4), in the undeformed state as manufactured, forms a level, disc-shaped component of constant thickness with the coil (3) arranged levelly in it.

3. Shock wave source according to Claim 1 or 2, **characterized in that** the membrane (2) is in the form of a deep-drawn metal cap.

4. Shock wave source according to one or more of Claims 1 to 3, **characterized in that** a propagation medium (5) in the form of a liquid or gel medium is bounded by a wall (6), which is in the form of a protective foil or skin.

5. Shock wave source according to one or more of Claims 1 to 4, **characterized in that** the housing (8) is in the form of a hard plastic part with shaped-on handle (10) and integrated release button (11), and preferably in the form of an injection-moulded part.

6. Shock wave source according to one or more of Claims 4 or 5, **characterized in that** the coil support (4), the membrane (2) and the wall (6) which bounds the propagation medium (5) externally are held with positive locking and removably in the housing (8).

## Revendications

1. Générateur d'onde de choc électromagnétique (1), doté d'une focalisation, en particulier une tête de thérapie guidée manuellement, d'une membrane électriquement conductrice, courbée spatialement (2), d'une bobine (3), excitable par impulsions de courant, adjacente à cette membrane, d'une connexion électrique commutable (11, 12, 13) entre la bobine (3) et une source d'alimentation électrique, d'un logement (8) recevant la membrane (2) et la bobine encastrée dans un support de bobine (4) recevant une contrainte de compression définie, le support de bobine (4) étant situé entre le logement et la membrane (2), ainsi que d'un milieu de conduction (5) adjacent à la membrane (2) et exerçant une pression sur cette dernière, **caractérisé en ce que** le support de bobine (4) est déformable et élastiquement compressible, **en ce que** le support de bobine (4) est installé à l'état comprimé et **en ce qu'**après le montage, la quantité incorporée du milieu de conduction (5) reste non influencée pendant l'état de fonctionnement.

2. Générateur d'onde de choc selon la revendication 1, **caractérisé en ce que** le support de bobine (4), à l'état non déformé déterminé par les conditions de fabrication, forme un élément de construction plan, en forme de disque et d'épaisseur constante, dans lequel la bobine est disposée de façon plane.

3. Générateur d'onde de choc selon la revendication 1 ou 2, **caractérisé en ce que** la membrane (2) est faite sous la forme d'une calotte métallique emboutie en profondeur.

4. Générateur d'onde de choc selon l'une ou plusieurs des revendications 1 à 3, **caractérisé en ce qu'**un milieu de conduction fait sous la forme d'un milieu liquide ou gélatineux (5) est limité vers l'extérieur par une paroi (6), laquelle est faite sous la forme d'une feuille de protection ou d'une pellicule ("Balg" en allemand).

5. Générateur d'onde de choc selon l'une ou plusieurs des revendications 1 à 4, **caractérisé en ce que** le logement (8) est fait sous la forme d'un élément en matière plastique rigide équipé d'une poignée façonnée (10) et d'un bouton de déclenchement intégré (11), de préférence sous forme d'élément moulé par injection.

6. Générateur d'onde de choc selon la revendication 4 ou 5, **caractérisé en ce que** le support de bobine (4), la membrane (2) et la paroi (6) limitant vers l'extérieur le milieu de conduction (5) sont maintenus dans le logement (8) par clabotage et sont démontables.
